# EUROPEAN PATENT SPECIFICATION

(11) **EP 1 757 263 B1**
(45) Date of publication and mention of the grant of the patent: **23.09.2009**
(21) Application number: 06025559.3
(22) Date of filing: 19.06.2000
(51) Int. Cl.: A61K 8/28, A61K 8/67, A61K 8/898, A61Q 15/00

(54) **Dermatic cosmetic material**
Kosmetische Zusammensetzung für die Haut
Composition cosmétique pour le traitement de la peau

(30) Priority: 24.06.1999 JP 17790499
(43) Date of publication of application: 28.02.2007
(62) Divisional of application: 00305170.3
(73) Proprietor: Shin-Etsu Chemical Co., Ltd., Chiyoda-ku, Tokyo 100-0004 (JP)
(72) Inventor: Sakuta, Koji c/o Silicone Electronic Res. Center, Matsuidamchi Usui-gun Gnma 379-0224 (JP)
(74) Representative: Wilson Gunn

(56) References cited:
- EP-A2- 0 501 791
- WO-A-98/43598
- US-A1- 5 412 004
- US-A1- 5 853 741

## Description

### FIELD OF THE INVENTION

The present invention relates to a dermatic cosmetic material in which is mixed a paste composition comprising a three-dimensionally cross-linked silicone polymer and a silicone oil. The invention relates to a skin-care cosmetic material in which vitamin C is dispersed homogeneously in a stable condition owing to the incorporation of the aforementioned paste composition.

### BACKGROUND OF THE INVENTION

Where it is intended that various vitamins be compounded in cosmetic materials for the purpose of furnishing nutrition to the skin, water-soluble vitamin C is difficult to disperse homogeneously into the cosmetic materials in a stabilized condition. This is because the water solution of vitamin C is liable to undergo oxidative decomposition, and so it is inferior in storage stability. Even when such cosmetic materials are prepared as emulsions of oil-in-water or water-in-oil type, their storage stability problem cannot be resolved. Therefore, cases are known where the cosmetic materials are prepared as nonaqueous compositions whose dispersion medium is alcohol. When the silicone oils as texture improver are added to such compositions, however, it is difficult to obtain homogeneous dispersions.

US-5853741 discloses cosmetic compositions which can stably store ascorbic acid, comprising a volatile siloxane and a cross linked non-emulsifying siloxane elastomer.

### SUMMARY OF THE INVENTION

The object of the invention is to provide nonaqueous dermatic cosmetic materials of homogeneous paste type, including a vitamin C-containing skin-care cosmetic material, which contain a lower alcohol and a silicone oil as a dispersion medium and has high storage stability.

As a result of our intensive studies for attaining the aforementioned object, it has been found that vitamin C-containing skin-care composition having high storage stability can be obtained by using as a substrate a silicone composition paste comprising (i) a silicone polymer having hydrophilic polyoxyalkylene groups, wherein polyoxyethylene moieties are comprised as defined in claim 1 and (ii) a silicone oil, thereby achieving the present invention.

Namely, the present invention provides a dermatic cosmetic material containing as a substrate a silicone composition paste comprising a silicone oil and a cross-linked silicone polymer having hydrophilic polyoxyalkylene groups in which polyoxyethylene moieties are comprised.

### DETAILED DESCRIPTION OF THE INVENTION

The present silicone composition paste is a composition prepared by dispersing homogeneously in a silicone oil a cross-linked silicone polymer having hydrophilic polyoxyalkylene groups which is produced by addition polymerization reaction between an organohydrogenpolysiloxane and a compound having terminal aliphatic unsaturated groups. The organohydrogenpolysiloxane usable in this addition polymerization reaction is a hydrophilic polyoxyalkylene group-containing organohydrogenpolysiloxane represented by formula, **R¹ₐR²_{b}H_{c}SiO_{(4-a-b)/2}** (1), an organohydrogenpolysiloxane represented by formula, **R¹ⱼHₖSiO_{(4-j-k)/2}** (2), or a mixture of these organohydrogenpolysiloxanes; while the compound having terminal aliphatic unsaturated groups usable in this addition polymerization reaction is a polyalkylene oxide represented by formula, **CₘH₂ₘ₋₁(C₂H₄O)ₚ (C₃H₆O)_{q} CₘH₂ₘ₋₁** (A), an organopolysiloxane represented by formula, **R¹_{d}R³ₑSiO_{(4-d-e)/2}** (B), or a mixture of these compounds. Moreover, at least either the organohydrogenpolysiloxane represented by formula (1) or the polyalkylene oxide represented by formula (A) is the essential component in the aforementioned addition polymerization reaction. In those formulae, R¹ represents an alkyl group containing 1 to 18 carbon atoms, an aryl group, an aralkyl group or a monovalent halogenated hydrocarbon group; R² represents an organic group represented by **CₙH₂ₙO (C₂H4O)_{f} (C₃H₆O) _{g}R⁴:** R³ represents a monovalent 2-10C hydrocarbon group having a terminal vinyl group; R⁴ represents a hydrogen atom, a 1-10C saturated organic group, or a group represented by -CO-R⁵; R⁵ represents a 1-5C saturated organic group; 1.0 ≤ a ≤2.5, 0.001≤ b ≤1.0, 0.00≤ c ≤2. 0, 1.0≤ d ≤3.0, 0.001≤ e ≤1.5, 1.0≤ j ≤3.0, 0.001≤ k ≤1.5; f and p are each an integer of from 2 to 200; g and q are each an integer of from 0 to 200; and m and n are each an integer of from 2 to 6. More specifically, the present silicone composition paste includes the silicone composition disclosed in Japanese Tokkai Hei 4-272932 (the term "Tokkai" as used herein means an "unexamined published patent application"), and the silicone composition disclosed in Japanese Tokkai Hei 5-140320 which is prepared by making a combination of proper compounds to synthesize a cross-linked silicone polymer having hydrophilic polyoxyalkylene groups, wherein one compound is selected from organohydrogenpolysiloxanes represented by formula (1), organohydrogenpolysiloxanes represented by formula (2) or mixtures thereof and the other compound is selected from polyalkylene oxides represented by formula (A), organopolysiloxanes represented by formula (B) or mixtures thereof, provided that at least either organohydrogenpolysiloxane of formula (1) or polyalkylene oxide of formula (A) is included in the combination, subjecting the combination to addition polymerization reaction in the presence of a low-viscosity silicone oil having a viscosity of no higher than 100 mm²/s at 25°C, a polyhydric alcohol or a mixture thereof, and then dispersing the thus prepared silicone polymer into a silicone oil.

In accordance with the invention, the silicone composition paste as mentioned above is employed as a substrate (Component (A)) and combined with the following Components (B) to (D), thereby preparing a dermatic cosmetic material such as a skin-care composition.

Examples of a lower alcohol usable as Component (B) include monohydric alcohols, such as ethanol and 2-propanol, and polyhydric alcohols, such as ethylene glycol, diethylene glycol, triethylene glycol, propylene glycol, dipropylene glycol, tripropylene glycol, isoproterenol and glycerine. Of these alcohols, water-soluble alcohols, especially ethanol and dipropylene glycol, are preferred in the invention.

Silicone oils usable as Component (C) are those having a viscosity of no higher than 100 mm²/s at 25°C, with examples including straight-chain or branched dimethylsilicone, methylphenylsilicone and fluorine-modified silicone. In the invention, silicone oils having a viscosity of no higher than 50 mm²/s are used to advantage. In particular, volatile dimethylsilicones having a boiling point of no higher than 250°C are preferred over the others.

In the case of preparing a skin-care composition containing vitamin C, the content of Component (B) is from 100 to 1,000 parts by weight per 100 parts by weight of Component (A), that of Component (C) is from 100 to 1,000 parts by weight per 100 parts by weight of Component (A) and that of Vitamin C be from 0.5 to 100 parts by weight per 100 parts by weight of Component (A).

When the content of Component (B) is lower than 100 parts by weight, the resultant composition is short of compatibility with vitamin C; while, when it is higher than 1,000 parts by weight, the resultant composition cannot does not feel comfortable. Therefore, the preferable content of Component (B) is from 100 to 500 parts by weight. As to Component (C), when its content is lower than 100 parts by weight, the resultant composition does not feel comfortable; while, when its content is increased beyond 1,000 parts by weight, the resultant composition is not in a paste state. Therefore, the preferable content of Component (C) is from 100 to 500 parts by weight.

When the content of vitamin C. is lower than 0.5 parts by weight, the resultant composition has little skin-care effect; while, when it is increased beyond 100 parts by weight, it becomes difficult to prepare a homogeneous dispersion. Therefore, the especially desirable content of vitamin C is from 0.5 to 50 parts by weight.

Besides the foregoing components, the ingredients usable for general skin-care cosmetics, such as oils including ester oil and hydrocarbon oil, higher alcohols including cetyl alcohol and stearyl alcohol, perfume and vitamins including vitamins A, B, D, E, F, K, L, T and U, may be mixed in the present skin-care composition.

The skin-care composition obtained has excellent storage stability and can give comfortable feelings to the users. In other words, the dermatic cosmetic materials according to the present invention have significant improvements over conventional ones.

Now, the present invention will be illustrated in greater detail by reference to the following examples. However, the invention should not be construed as being limited to these examples. Additionally, the silicone composition paste used as a substrate is prepared using the silicone polymer synthesized in the following Synthesis Example 1.

### SYNTHESIS EXAMPLE 1

In a reaction vessel were placed 100 g of organohydrogenpolysiloxanes represented by the following average structural formula, 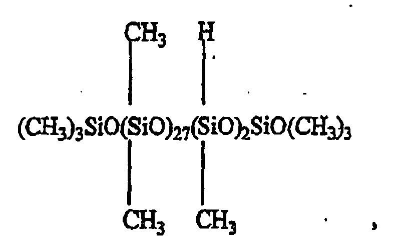 103.0 g of ethanol, 23.6 g of polyoxyalkylene represented by the following average structural formula,

CH₂=CHCH₂ (C₂H₄O)₁₀ CH₂CH=CH₂,

and 0.3 g of a 3 weight % ethanol solution of chloroplatinic acid. The contents in the reaction vessel were stirred for 2 hours while keeping the temperature thereof at 70-80°f, and then the solvent was removed under reduced pressure. Thus, a silicone polymer was obtained.

A 100 parts by weight portion of the silicone polymer thus obtained was mixed with 300 parts by weight of dimethylpolysiloxane having the viscosity of 6 mm²/s at 25°C, and further kneaded by means of a three-rod roll mill to prepare a silicone composition in a paste state (Silicone Composition No. 1).

Another 100 parts by weight portion of the, silicone polymer obtained was mixed with 400 parts by weight of decamethylcyclopentasiloxane, and further kneaded with a three-rod roll mill to prepare a silicone composition in a paste state (Silicone Composition No. 2).

### EXAMPLE 1 TO 3

### Skin-care Compositions containing vitamin C:

Vitamin C-containing skin-care compositions constituted of the ingredients shown in Table 1 were prepared.

**Table 1**

| Ingredients mixed | Proportion of Ingredients mixed (weight %) | | |
|---|---|---|---|
| | Example 1 | Example 2 | Example 3 |
| Silicone Composition 1 | 20.0 | 0 | 30.0 |
| Silicone Composition 2 | 0 | 30.0 | 0 |
| Decamethylcyclopenta-siloxane | 40.0.0 | 30.0 | 97.0 |
| Dimethylpolysiloxane (viscosity: 6 mm²/s) | 0 | 5.0 | 0 |
| Dipropylene glycol | 38.0 | 0 | 20.0 |
| 1,3-butylene glycol | 0 | 32.0 | 0 |
| Cetyl alcohol | 0 | 1.0 | 0 |
| Vitamin C | 2.0 | 1.0 | 2.0 |
| Vitamin E | 0 | 1.0 | 1.0 |

Each of the thus prepared vitamin C-containing skin-care compositions spread smoothly, gave a refreshing feeling, had neither tacky nor oily feel, and caused no change by temperature and aging. In other words, the skin-care compositions according to the invention had excellent usability and stability despite the incorporation of vitamin C therein.

## Claims

1. A nonaqueous dermatic cosmetic material; comprising
(A) 100 parts by weight of the silicone composition paste comprising (i) a cross-linked silicone polymer having polyoxyethylene groups wherein the cross-linked silicone polymer is a polymer produced by carrying out addition polymerization reaction of a polyoxyethylene group-containing organohydrogenpolysiloxane, an organohydrogenpolysiloxane or a mixture thereof with a hydrophilic polyalkylene oxide having terminal aliphatic unsaturated groups, or a mixture of the hydrophilic polyalkylene oxide and an organopolysiloxane having terminal aliphatic unsaturated groups, and (ii) a silicone oil,
(B) 100 to 1,000 parts by weight per 100 parts of component (A) of a lower alcohol
(C) 100 to 1,000 parts by weight per 100 parts of component (A) of a silicone oil having a viscosity of at most 100 mm²/s at 25°C, and
(D) 0.5 to 100 parts by weight per 100 parts of component (A) C.

## Patentansprüche

1. Nicht-wässriges hautkosmetisches Material; umfassend
(A) 100 Gewichtsteile einer Siliconzusammensetzungspaste umfassend (i) ein vernetztes Siliconpolymer mit Polyoxyethylengruppen, wobei das vernetzte Siliconpolymer ein Polymer ist, das hergestellt ist durch Ausführen einer Additionspolymerisationsreaktion von einem eine Polyoxyethylengruppe enthaltenden Organhydrogenpolysiloxan, einem Organohydrogenpolysiloxan oder einer Mischung davon mit einem hydrophilen Polyalkylenoxid, das endständige aliphatische ungesättigte Gruppen aufweist, oder einer Mischung des hydrophilen Polyalkylenoxids und eines Organopolysiloxans, das endständige aliphatische ungesättigte Gruppen aufweist, und (ii) ein Silikonöl
(B) 100 bis 1000 Gewichtsteile eines niederen Alkohols pro 100 Teile von Komponente (A),
(C) 100 bis 1000 Gewichtsteile eines Siliconöls mit einer Viskosität von höchstens 100 mm²/s bei 25 °C pro 100 Teile von Komponente (A), und
(D) 0,5 bis 100 Gewichtsteile Vitamin C pro 100 Teile von Komponente (A).

## Revendications

1. Matière cosmétique dermatologique non aqueuse, comprenant :
(A) 100 parties en poids d'une pâte d'une composition de silicone comprenant (i) un polymère de silicone réticulé ayant des groupes polyoxyéthylène dans laquelle le polymère de silicone réticulé est un polymère produit en menant une réaction de polymérisation par addition d'un organohydrogénopolysiloxane contenant un groupe polyoxyéthylène, un organohydrogénopolysiloxane ou un mélange de celui-ci avec un oxyde de polyalkylène hydrophile ayant des groupes insaturés aliphatiques terminaux, ou un mélange de l'oxyde de polyalkylène hydrophile et d'un organopolysiloxane ayant des groupes insaturés aliphatiques terminaux, et (ii) une huile de silicone,
(B) 100 à 1000 parties en poids pour 100 parties de composant (A) d'un alcool inférieur,
(C) 100 à 1000 parties en poids pour 100 parties de composant (A) d'une huile de silicone ayant une viscosité d'au plus 100 mm²/s à 25 °C et
(D) 0,5 à 100 parties en poids pour 100 parties de composant (A) de vitamine C.
